# EUROPÄISCHE PATENTSCHRIFT

(11) **EP 1 112 242 B1**
(45) Veröffentlichungstag und Bekanntmachung des Hinweises auf die Patenterteilung: **04.06.2003**
(21) Anmeldenummer: 99950470.7
(22) Anmeldetag: 30.07.1999
(51) Int. Cl.: C07C 6/00

(54) **KONTINUIERLICHES VERFAHREN UND WÄRMEPUMPENVORRICHTUNG ZUR ANREICHERUNG FLÜSSIGER NIEDRIG KONZENTRIERTER REAKTIONSGEMISCHE**
CONTINUOUS METHOD AND HEAT PUMP DEVICE FOR ENRICHING LIQUID LOW-CONCENTRATED REACTION MIXTURES
PROCEDE CONTINU ET DISPOSITIF DE THERMOPOMPE PERMETTANT L'ENRICHISSEMENT DE MELANGES REACTIONNELS LIQUIDES DE FAIBLE CONCENTRATION

(30) Priorität: 04.08.1998 DE 19835203
(43) Veröffentlichungstag der Anmeldung: 04.07.2001
(73) Patentinhaber: HAARMANN & REIMER GMBH, 37603 Holzminden (DE)
(72) Erfinder: BRABAND, Jürgen, D-04275 Leipzig (DE); MÜLLER, Peter, D-04175 Leipzig (DE); BERNHARDT, Rüdiger, D-06774 Plodda (DE); OTTO, Andreas, D-06800 Je nitz (DE)
(74) Vertreter: Eisenführ, Speiser & Partner
(86) Internationale Anmeldenummer: DE9902347
(87) Internationale Veröffentlichungsnummer: WO00007967

(56) Entgegenhaltungen:
- EP-A- 0 343 437

## Beschreibung

Die Erfindung betrifft ein kontinuierliches Verfahren und eine Wärmepumpenvorrichtung zur Anreicherung von bei der Herstellung von Cycloalkandienen mittels katalytischer Metathese aus cyclischen aliphatischen Alkenen und Cyclooligomeren in organischen Reaktionsmedien anfallenden niedrig konzentrierten Reaktionsgemischen.

Wertvolle Moschusriechstoffe werden synthetisch aus Cycloalkandienen mit einer Ringgröße zwischen 12 und 18 C-Atomen bevorzugt mit 16 C-Atomen, dem Cyclohexadekadien, hergestellt.

Die Herstellung von Cycloalkandienen insbesondere von Cyclohexadekadien wird in GB-A 1105565 (1), EP-A 0192333 (2), EP-B 0343437 (3) und DE OS 197 27 256 (4) beschrieben. Nach den beschriebenen Verfahren erhält man Cycloalkandiene, ausgehend von den cyclischen Alkenen mit 6 - 9 C-Atomen (1,2) oder Cyclopolyoctenylen (3) mit einem Polymerisationsgrad größer oder gleich 3, wenn die Ausgangsstoffe einer Methathesenreaktion in Flüssigphase an einem Trägerkatalysator auf Basis Re₂O₇/Al₂O₃ /1,2) oder modifizierten Trägerkatalysator (3,4) umgesetzt werden.

In (2) wird erstmals darauf hingewiesen, daß die Selektivität der Zielprodukte und damit die Ausbeute erhöhbar ist, wenn die Metathesereaktion das Dimerisierungsstadium nicht überschreitet. Dies gelingt, in dem man mit hochverdünnten Lösungen der Ausgangsprodukte (0,01 bis 0,05 molar) arbeitet.

Als Metathese-inerte Lösungsmittel zur Verdünnung der Ausgangsstoffe werden aliphatische Alkane wie Pentan, Hexan, Heptan, Cyclopentan, Cyclohexan, Petroläther, chlorierte Kohlenwasserstoffe wie Methylenchlorid, Chloroform, Tetrachlorkohlenstoff oder Aromaten wie Chlorbenzol, m-Dichlorbenzol eingesetzt (2,3,4).

Nach dem Durchströmen des Katalysatorbettes liegt ein niedrig konzentriertes Reaktionsgemisch im organischen Lösungsmittel vor, das Zielprodukt, Nebenprodukte und unumgesetzte Ausgangsstoffe enthält.

Nach (2,3,4) wird das den Reaktor verlassende Reaktionsgemisch einer Destillationsvorrichtung aufgegeben und in seine Komponenten getrennt, wobei das Zielprodukt als relativ hochsiedende Fraktion anfällt und das niedrig siedende Lösungsmittel sowie ggf. unumgesetzte Ausgangsstoffe im Kreislauf geführt und nach Beschickung mit neuen Ausgangsprodukten in den Reaktor zurückgeleitet werden.

Zur Erhöhung der Selektivität der Metathesereaktion in Richtung Zielprodukte Cycloalkandiene mit 12 - 18 C-Atomen ist das Arbeiten in hochverdünnten Lösungen mit Verhältnissen Ausgangsstoff:Lösungsmittel 1:1300 zwingend erforderlich.

In diesem Sachverhalt liegt jedoch auch der wesentliche Nachteil dieser Verfahren, der bisher die technische Realisierung der Verfahren erheblich einschränkte oder verhinderte. Die Auftrennung der Reaktionslösung in Zielprodukt und Lösungsmittel mittels der üblichen Blasendestillation ist mit hohem Energieaufwand für die Verdampfung des Lösungsmittels verbunden und belastet die Kosten für die Herstellung des Zielproduktes maßgeblich und damit auch den Preis des synthetischen Riechstoffes.

Es besteht deshalb die Aufgabe, ein Verfahren zu finden, mit dem der energetische Aufwand für die Auftrennung der Reaktionslösung in Zielprodukt, Nebenprodukte und organisches Reaktionsmedium (Lösungsmittel) wesentlich gesenkt und das organische Reaktionsmedium gleichzeitig im Kreislauf geführt wird.

Es wurde nun ein Verfahren gefunden, welches oben angeführte Aufgabe unter Ausnutzung des Prinzipes der Wärmepumpe löst, in dem das Wärmeniveau des organischen Reaktionsmediums fortlaufend in einem engen Temperaturbereich angehoben und wieder abgesenkt wird und kein ständiges externes Verdampfen und Kondensieren erfolgen muß. Dieses Ziel wird über eine geeignete Schaltung von Wärmeaustauschern in Kombination mit einem Brüdenkompressor erreicht. Dabei wird über den Brüdenkompressor elektrische Energie in Wärme umgewandelt, mit der das organische Reaktionsmedium verdichtet und erwärmt wird. Die im Brüdenkompressor aufgenommene Wärme wird direkt zur Verdampfung des organischen Reaktionsmediums aus dem vorliegenden Reaktionsgemisch im Wärmetauscher genutzt, so daß ein neuer Zyklus beginnt.

Das Verfahren, mit dem vorstehende Aufgabe gelöst wird, ist durch die in Ansprüchen 1 - 4 aufgeführten Merkmale gekennzeichnet, welches mittels der im Anspruch 5 beschriebenen Vorrichtung durchführbar ist.

Die Temperatur T 1, mit der das niedrig konzentrierte Reaktionsgemisch in seinem organischen Reaktionsmedium vom Reaktor über die Zuführungsleitung (1) in den ein- oder mehrkammerigen Verdampfer (2) kontinuierlich eingeleitet wird, wird von Reaktionsbedingungen der katalytischen Metathesereaktion bestimmt, insbesondere von der Temperaturbeständigkeit des Katalysators, der Selektivität der katalytischen Reaktion hinsichtlich Umsatz der Ausgangsstoffe zum Zielprodukt in Abhängigkeit von der Temperatur und dem in Verbindung damit eingesetzten organischen Reaktionsmedium. Die Kochpunkte der in Anspruch 2 aufgeführten aliphatischen, cyclisch aliphatischen und chlorierten Kohlenwasserstoffe liegen im Bereich zwischen 30° C und 120° C. Mit dem Ziel, den energetischen Aufwand für die Anreicherung des Reaktionsgemisches im organischen Reaktionsmedium so niedrig wie möglich zu halten, ist das organische Reaktionsmedium so zu wählen, daß dessen Kochpunkt nur wenige ° C über der Reaktionstemperatur der Ausgangsstoffe am Katalysator liegt.

Das organische Reaktionsmedium wird deshalb so gewählt, daß die Temperaturdifferenz zwischen der Eingangstemperatur T 1 der Reaktionslösung im Verdampfer und dem Kochpunkt des zu verdampfenden organischen Reaktionsmediums 5 K nicht übersteigt.

Mit der Anreicherung des Reaktionsgemisches im organischen Reaktionsmedium tritt eine Kochpunkterhöhung des eingesetzten Lösungsmittels ein. Wie Siedepunktskurven zu entnehmen ist, steigt der Kochpunkt des Lösungsmittels signifikant an, wenn das Reaktionsgemisch auf mehr als 50 Gew.% im organischen Reaktionsmedium angereichert wird. In Abhängigkeit von den konkreten Verfahrensbedingungen ist der energetische Aufwand am niedrigsten, wenn das Reaktionsgemisch im Verdampfer auf 30 - 50 Gew.% im organischen Reaktionsmedium angereichert wird.

Aus dem Verdampfer werden die Dämpfe des organischen Reaktionsmediums abgesaugt und verdichtet, wobei die Temperatur auf T 2 steigt. Die Druckdifferenz zwischen Saug- und Druckseite des Kompressors wird so gewählt, daß die Temperatur T 2 5 - 12 K über T 1 liegt. Dies wird erreicht mit einer Druckdifferenz von 0,25 - 1 bar. Der auf die Temperatur T 2 verdichtete Dampf des organischen Reaktionsmediums wird in die Wärmetauscher des Verdampfers zurückgeführt und überträgt die aufgenommene Wärmeenergie auf das mit der Temperatur T 1 im Verdampfer vorliegende organische Reaktionsmedium, welches verdampft. Am kostengünstigsten verläuft dieser Wärmeaustausch, wenn die Temperaturdifferenz zwischen T 1 und T 2 5 K nicht unterschreitet, bevorzugt jedoch zwischen 8 und 12 K beträgt.

Unter Anwendung des beschriebenen Verfahrens werden niedrig konzentrierte flüssige Reaktionsgemische mit einem Gehalt von mindestens 0,1 Gew.% im organischen Reaktionsmedium unter Senkung des energetischen Aufwandes auf einen Gehalt von 30 - 50 Gew.% im Verdampfer angereichert.

Das auf diesen Wertstoffgehalt angereicherte Reaktionsgemisch wird über die Leitung (9) kontinuierlich vom Verdampfer abgezogen und der Destillationsanlage (10) zur weiteren Auftrennung in organisches Reaktionsmedium, unumgesetzte Ausgangsstoffe, Nebenprodukte und Zielprodukt zugeführt. Organisches Reaktionsmedium und unumgesetzte Ausgangsstoffe werden im Behälter (8) mit dem aus dem Wärmetauscher (6) des Verdampfers (2) kondensierten organischen Reaktionsmedium wieder vereinigt und mit Ausgangsstoffen versetzt in den Reaktor eingetragen.

Mit dem erfindungsgemäßen Verfahren kann der Energieverbrauch für die Verdampfung des organischen Reaktionsmediums um ca. 80 % und die Kosten um ca. 30 % gesenkt werden. An nachfolgendem Ausführungsbeispiel wird das Verfahren praktisch erläutert.

### Ausführungsbeispiel:

Reaktionsgemisch aus dem Reaktor, dessen Wertstoffgehalt 0,34 Gew.% beträgt, bestehend aus 2 Gew.% Cycloocten, 30 Gew.% Cyclohexadecadien und 60 Gew.% Oligomere in n-Hexan, wird in einen Mehrkammerverdampfer kontinuierlich eingetragen. Bei einer Verdampferleistung von 17.000 kg/h n-Hexan wird der Gehalt des Wertstoffgemisches im Verdampfer auf 35 Gew.% erhöht.

Die Eingangstemperatur beträgt 67° C. Der Kochpunkt von n-Hexan ist 68,5° C. Durch die Aufkonzentrierung des Wertstoffgemisches im n-Hexan bis auf 35 Gew.% erhöht sich der Siedepunkt des Gemisches im Verdampfer auf 72° C T 1. Der n-Hexandampf wird mittels einer Druckdifferenz von 0,41 bar im Kompressor verdichtet, wobei sich die Temperatur auf 81° C (T 2) erhöht. T 2 - T 1 beträgt somit 9 K.

Der verdichtete n-Hexandampf wird durch die Wärmetauscher des Verdampfers geleitet und gibt Wärmeenergie an n-Hexan der Temperatur T 1 ab. Kondensiertes n-Hexan gelangt aus dem Wärmetauscher nachfolgend in den Mischungsbehälter.

Bei o. a. Verdampferleistung an n-Hexan werden kontinuierlich/h 165 kg auf 35 Gew.% angereichertes Reaktionsgemisch vom Verdampfer abgezogen. Der Anteil des Wertstoffgemisches beträgt 57,5 kg. Der Energieverbrauch liegt bei ca. 100 kWh.

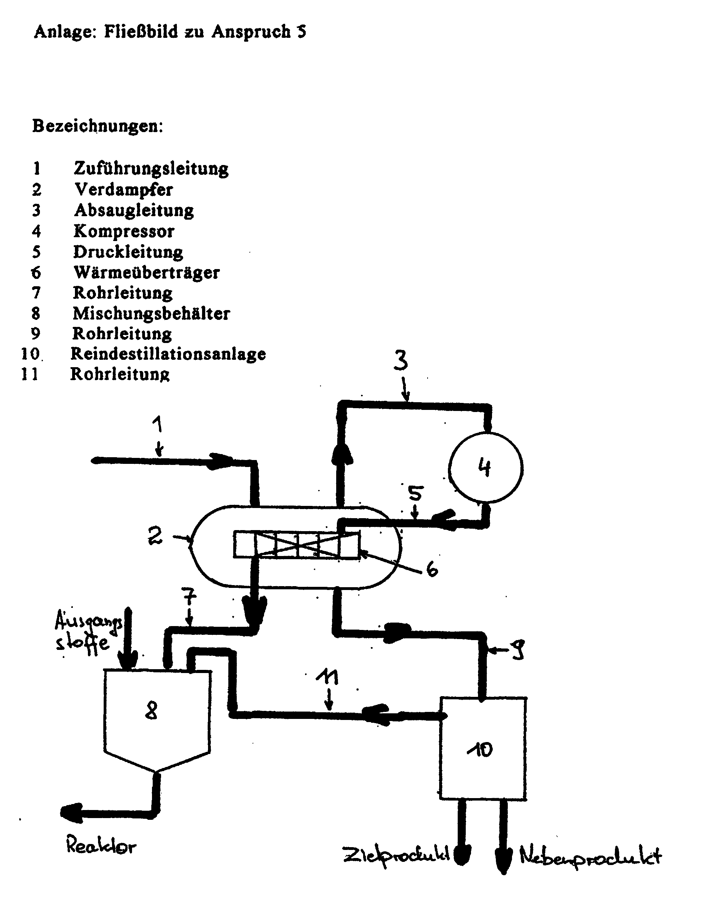

## Patentansprüche

1. Kontinuierliches Verfahren zur Anreicherung der bei der katalytischen Metathese von Cycloalkandienen aus Gemischen von cyclischen aliphatischen Alkenen und Cyclooligomeren in flüssigen organischen Reaktionsmedien, bei denen es sich um aliphatische, cyclische aliphatische und chlorierte Kohlenwasserstoffe handelt, in einer Konzentration von mindestens 0,1 Gew.% anfallenden Reaktionsgemische, bestehend aus unumgesetzten Ausgangsstoffen, dem Zielprodukt und Nebenprodukten, wobei die flüssigen organischen Reaktionsmedien und Anteile nicht umgesetzter Ausgangsstoffe im Kreislauf geführt, zur Nutzung als Wärmeträger bei einer Temperaturdifferenz zwischen T 1 und T 2 von mindestens 5 K verdampft und verdichtet werden und kondensiert einem Mischungsbehälter zur Einstellung der verfahrensbedingten Konzentrationsverhältnisse der Ausgangsstoffe zugeführt werden, umfassend die Verfahrensmaßnahmen.
1a. Einbringen der kontinuierlich aus dem Reaktionsbehälter abgezogenen niedrig konzentrierten Reaktionsgemische mit der Temperatur T 1 in einen ein- oder mehrkammerigen Verdampfer.
1b. Absaugen der Dämpfe des organischen Reaktionsmediums aus dem Verdampfer.
1c. Verdichtung der Dämpfe in einem Brüdenkompressor mittels zugeführter Elektroenergie.
1d. Rückführung der verdichteten Dämpfe mit der Temperatur T 2 in den Wärmeüberträger des Verdampfers.
1e. Wärmeübertragung im Verdampfer zwischen dem niedrig konzentrierten Reaktionsgemisch der Temperatur T 1 und dem zurückgeführten verdichteten organischen Reaktionsmedium der Temperatur T 2.
1f. Kontinuierliche Rückführung des kondensierten organischen Reaktionsmediums aus dem Wärmeüberträger des Verdampfers in einen Mischungsbehälter zur Einstellung der verfahrensbedingten Konzentrationsverhältnisse.
1g. Kontinuierlicher Abzug des im Reaktionsmedium auf einen Gehalt von mindestens 30 Gew.% angereicherten Reaktionsgemisches aus dem Verdampfer, bestehend aus dem Zielprodukt, Nebenprodukte, Ausgangsstoffe und organischem Reaktionsmedium.
1h. Zuführung des angereicherten Reaktionsgemisches einer Reindestillationsanlage zum Abdestillieren des Restgehaltes des organischen Reaktionsmediums sowie zum Auftrennen des Reaktionsgemisches in Ziel-, Nebenprodukte und Ausgangsstoff.
1i. Rückführung des kondensierten organischen Reaktionsmediums und der Ausgangsstoffe in den Mischungsbehälter nach 1f.

2. Verfahren nach Anspruch 1, **dadurch gekennzeichnet, daß** es sich bei den aliphatischen, cyclischen aliphatischen und chlorierten Kohlenwasserstoffen um Pentan, Hexan, Heptan, Cyclopentan, Cyclohexan, Cycloheptan, Methylenchlorid, Chloroform, Tetrachlorkohlenstoff und Petroläther handelt.

3. Verfahren nach Anspruch 1 und 2, **dadurch gekennzeichnet, daß** die Temperatur T 1 den Kochpunkt des flüssigen organischen Reaktionsmediums nicht mehr als 5' K unterschreitet.

4. Verfahren nach Anspruch 1 bis 3, **dadurch gekennzeichnet, daß** die Temperaturdifferenz zwischen T 1 und T 2 bevorzugt 8 - 12' K beträgt.

5. Vorrichtung zur Durchführung des nach Anspruch 1 - 4 beanspruchten Verfahrens bestehend aus
5a. einer Zuführungsleitung (1) für das niedrig konzentrierte Reaktionsgemisch im flüssigen organischen Reaktionsmedium.
5b. einem ein- oder mehrkammerigen Verdampfer (2) mit einem Wärmeüberträger (6), in dem das flüssige organische Reaktionsmedium verdampft und die anderen Bestandteile des Reaktionsgemisches (Zielprodukt, Nebenprodukte, Ausgangsstoffe) angereichert werden.
5c. einer Absaugleitung (3) mit der der Dampf des organischen Reaktionsmediums zum Kompressor (4) gefördert wird.
5d. einem Kompressor (4), in dem der Dampf des organischen Reaktionsmediums verdichtet und auf Temperatur T 2 erwärmt wird.
5e. einer Druckleitung (5) zum Transport des verdichteten und erwärmten organischen Reaktionsmediums zum Wärmeüberträger (6).
5f. einem Wärmeüberträger (6) im Verdampfer (2) zur Wärmeübertragung zwischen dem flüssigen organischen Reaktionsmediums sowie dem Reaktionsgemisch der Temperatur T 1 und dem verdichteten organischen Reaktionsmedium der Temperatur T 2.
5g. einer Rohrleitung (7) zum Transport des kondensierten organischen Reaktionsmediums vom Wärmeüberträger (6) zum Mischungsbehälter (8).
5h. einer Rohrleitung (9) zur Förderung des angereicherten Reaktionsgemisches vom Verdampfer (2) zur Reindestillationsanlage (10).
5i. einer Reindestillationsanlage (10) zur Trennung des angereicherten Reaktionsgemisches in Zielprodukt, Nebenprodukte, Ausgangsstoffe und organischen Reaktionsmedium.
5j. einer Rohrleitung (11) zur Förderung des flüssigen organischen Reaktionsmediums von der Destillationsanlage (10) zum Mischungsbehälter (8).

## Claims

1. A continuous process for enriching the reaction mixtures which occur in a concentration of at least 0.1 % by weight on catalytic metathesis of cycloalkanedienes from mixtures of cyclic aliphatic alkanes and cyclooligomers in liquid organic reaction media, said media comprising aliphatic, cyclic aliphatic and chlorinated hydrocarbons, said reaction mixtures consisting of unreacted starting substances, the target product and secondary products, wherein the liquid organic reaction media and fractions of unreacted starting substances are circulated, are vaporised and compressed for use as a heat-transfer medium at a temperature difference between T1 and T2 of at least 5 K and, once condensed, are supplied to a mixing vessel in order to adjust the concentration ratios of the starting substances required by the process, said continuous process comprising the processing measures:
1a. introduction of the low-concentration reaction mixtures continuously withdrawn from the reaction vessel at temperature T1 into a single or multiple chamber vaporiser.
1b. exhausting the vapours of the organic reaction medium from the vaporiser.
1c. compression of the vapours in a vapour compressor by means of supplied electrical energy.
1d. returning the compressed vapours at temperature T2 to the vaporiser heat exchanger.
1e. heat exchange in the vaporiser between the low-concentration reaction mixture at temperature T1 and the returned, compressed organic reaction medium at temperature T2.
1f. continuous return of the condensed organic reaction medium from the vaporiser heat exchanger to a mixing vessel to establish the concentration ratios required by the process.
1g. continuous withdrawal of the reaction mixture, which has been enriched to a content of at least 30 % by weight in the reaction medium, from the vaporiser, said mixture consisting of the target product, secondary products, starting substances and organic reaction medium.
1h. introduction of the enriched reaction mixture into a purifying distillation unit to distil off the residual content of the organic reaction medium and to separate the reaction mixture into target product, secondary products and starting substance.
1i. return of the condensed organic reaction medium and the starting substances to the mixing vessel according to 1f.

2. A process according to claim 1, **characterised in that** the aliphatic, cyclic aliphatic and chlorinated hydrocarbons comprise pentane, hexane, heptane, cyclopentane, cyclohexane, cycloheptane, methylene chloride, chloroform, carbon tetrachloride and petroleum ether.

3. A process according to claims 1 and 2, **characterised in that** the temperature T1 is no more than 5 K below the boiling point of the liquid organic reaction medium.

4. A process according to claims 1 to 3, **characterised in that** the temperature difference between T1 and T2 is preferably 8-12 K.

5. An apparatus for carrying out the process claimed according to claims 1 to 4 consisting of
5a. a supply line (1) for the low-concentration reaction mixture in the liquid organic reaction medium.
5b. a single or multiple chamber vaporiser (2) with a heat exchanger (6), in which the liquid organic reaction medium is vaporised and the other constituents of the reaction mixture (target product, secondary products, starting substances) are enriched.
5c. an exhaust line (3) with which the vapour of the organic reaction medium is conveyed to the compressor (4).
5d. a compressor (4), in which the vapour of the organic reaction medium is compressed and heated to temperature T2.
5e. a pressure line (5) to transport the compressed and heated organic reaction medium to the heat exchanger (6).
5f. a heat exchanger (6) in the vaporiser (2) for heat exchange between the liquid organic reaction medium and the reaction mixture at temperature T1 and the compressed organic reaction medium at temperature T2.
5g. a pipe (7) to transport the condensed organic reaction medium from the heat exchanger (6) to the mixing vessel (8).
5h. a pipe (9) to convey the enriched reaction mixture from the vaporiser (2) to the purifying distillation unit (10).
5i. a purifying distillation unit (10) for separating the enriched reaction mixture into target product, secondary products, starting substances and organic reaction medium.
5j. a pipe (11) to convey the liquid organic reaction medium from the distillation unit (10) to the mixing vessel (8).

## Revendications

1. Procédé continu pour l'enrichissement de mélanges réactionnels produits dans une concentration d'au moins 0,1% en poids, lors de la métathèse catalytique de cycloalcanediènes à partir de mélanges d'alcènes aliphatiques cycliques et de cyclo-oligomères dans des milieux réactionnels organiques liquides, pour lesquels il s'agit d'hydrocarbures aliphatiques, cycliques aliphatiques et chlorés, lesquels mélanges réactionnels sont constitués de matières de départ n'ayant pas réagi, du produit ciblé et de produits secondaires, les milieux réactionnels organiques liquides et les parts en matières de départ n'ayant pas réagi étant remis en circulation, étant évaporés et concentrés pour une utilisation comme porteur de chaleur pour une différence de température entre T1 et T 2 d'au moins 5 K et étant introduits à l'état condensé dans un récipient de mélange pour l'ajustement des rapports de concentration selon le procédé des matières de départ, comprenant les mesures du procédé.
1a. Introduction des mélanges réactionnels faiblement concentrés soutirés en continu du récipient de la réaction avec la température T 1 dans un évaporateur à une ou plusieurs chambres.
1b. Aspiration des vapeurs du milieu réactionnel organique à partir de l'évaporateur.
1c. Concentration des vapeurs dans un compresseur de fumées au moyen d'une énergie électrique apportée.
1d. Recyclage des vapeurs concentrées avec la température T 2 dans le dispositif de transfert thermique de l'évaporateur.
1e. Transfert thermique dans l'évaporateur entre le mélange réactionnel faiblement concentré de la température T1 et le milieu réactionnel organique concentré renvoyé de la température T 2.
1f. Recyclage continu du milieu réactionnel organique condensé provenant du dispositif de transfert thermique de l'évaporateur dans un récipient de mélange pour l'ajustement des rapports de concentration selon le procédé.
1g. Soutirage continu du mélange réactionnel enrichi dans le milieu réactionnel jusqu'à une teneur d'au moins 30 % en poids à partir de l'évaporateur, mélange réactionnel constitué du produit ciblé, de produits secondaires, de matières de départ et de milieu réactionnel organique.
1h. Introduction du mélange réactionnel enrichi dans une installation de distillation de purification pour l'élimination par distillation de la teneur résiduelle du milieu réactionnel organique ainsi que pour la séparation du mélange réactionnel en produit ciblé, produits secondaires et matières de départ.
1i. Recyclage du milieu réactionnel organique condensé et des matières de départ dans le récipient de mélange selon 1f.

2. Procédé selon la revendication 1, **caractérisé en ce qu'**il s'agit pour les hydrocarbures aliphatiques, cycliques aliphatiques et chlorés du pentane, de l'hexane, de l'heptane, du cyclopentane, du cyclohexane, du cycloheptane, du chlorure de méthylène, du chloroforme, du tétrachlorocarbone et d'éther de pétrole.

3. Procédé selon les revendications 1 et 2, **caractérisé en ce que** la température T1 est inférieure au point d'ébullition du mélange réactionnel organique liquide d'au plus 5 K.

4. Procédé selon les revendications 1 à 3, **caractérisé en ce que** la différence de température entre T 1 et T 2 est de préférence de 8-12 K.

5. Dispositif pour la réalisation du procédé revendiqué dans les revendications 1-4 constitué de
5a. Une canalisation d'introduction (1) pour le mélange réactionnel faiblement concentré dans un milieu réactionnel organique liquide.
5b. Un évaporateur à une ou plusieurs chambres (2) avec un dispositif de transfert de chaleur (6), dans lequel le milieu réactionnel organique liquide est évaporé et les autres constituants du mélange réactionnel (produit ciblé, produits secondaires, matières de départ) sont enrichis.
5c. Une canalisation d'aspiration (3) avec laquelle les vapeurs du milieu réactionnel organique sont transportées jusqu'au compresseur (4).
5d. Un compresseur (4) dans lequel les vapeurs du milieu réactionnel organique sont concentrées et chauffées jusqu'à la température T 2.
5e. Une canalisation sous pression (5) pour le transport du milieu réactionnel organique comprimé et chauffé jusqu'au dispositif de transfert de chaleur (6).
5f. Un dispositif de transfert de chaleur (6) dans l'évaporateur (2) pour le transfert thermique entre le milieu réactionnel organique liquide ainsi que le mélange réactionnel de la température T1 et le milieu réactionnel organique concentré de la température T 2.
5g. Une canalisation (7) pour le transport du milieu réactionnel organique condensé du dispositif de transfert de chaleur (6) vers le récipient de mélange (8).
5h. Une canalisation (9) pour le transport du mélange réactionnel enrichi de l'évaporateur (2) vers l'installation de distillation de purification (10).
5i. Une installation de distillation de purification (10) pour la séparation du mélange réactionnel enrichi en produit ciblé, produits secondaires, matières de départ et milieu réactionnel organique.
5j. Une canalisation (11) pour le transport du milieu réactionnel organique liquide de l'installation de distillation (10) vers le récipient de mélange (8).
